# EUROPEAN PATENT SPECIFICATION

(11) **EP 0 727 219 B1**
(45) Date of publication and mention of the grant of the patent: **16.06.2004**
(21) Application number: 96300884.2
(22) Date of filing: 09.02.1996
(51) Int. Cl.: A61K 33/00

(54) **Medical gas mixture**
Medizinische Gasmischungen
Mélange de gaz médical

(30) Priority: 16.02.1995 GB 9502987
(43) Date of publication of application: 21.08.1996
(73) Proprietor: The BOC Group plc, Windlesham Surrey GU20 6HJ (GB)
(72) Inventor: Garrett, Michael Ernest, Woking, Surrey (GB)
(74) Representative: Bousfield, Roger James

(56) References cited:
- EP-A- 0 523 315
- GB-A- 1 396 772
- US-A- 4 554 916
- FISHER HK ET AL: "Site of action of inhaled 6 per cent carbon dioxide in the lungs of asthmatic subjects before and after exercise." AM REV RESPIR DIS, NOV 1976, 114 (5) P861-70, UNITED STATES, XP002074495
- GLUCK EH ET AL: "Helium-oxygen mixtures in intubated patients with status asthmaticus and respiratory acidosis." CHEST, SEP 1990, 98 (3) P693-8, UNITED STATES, XP002074496
- BARTHELEMY L ET AL: "Intérêt médical des mélanges respiratoires Oxygène-Hélium (Héliox)" REV PNEUMOL CLIN, 1993, 49 (2) P92-8, FRANCE, XP002074497
- CHOUX C ET AL: "Mélange hélium-oxygène. Prise en charge préhospitalière des asthmes aigus graves. tude prostective: SAMU 69 (october 1991-décember 1992)" URGENCES MEDICALES, 13 (6). 1994. 265-266., XP002074498

## Description

This invention relates to medical gas mixtures and, more particularly, to such mixtures for use in the treatment of chronic obstructive airway disease, asthma and various other medical conditions.

During an acute asthma attack, there is a marked exacerbation of the underlying inflammation in the small and medium sized airways in the lungs. The lining of the airways becomes oedematous, and the surrounding smooth muscle contracts. The transmission of air becomes restricted and turbulent resulting in ventilation/perfusion (V/Q) mismatching and hypoxaemia.

Acute asthma attacks are often initially treated with an inhaled beta adrenergic agonist that simulates the β-receptors on airway smooth muscles causing bronchodilatation. However, β-agonists do not treat the underlying inflammation and as the asthma attack develops, the β-receptors tend to become refractory to β-agonist medication.

Acute asthma causes hypoxaemia and the rational treatment of hypoxaemia is with oxygen. Hypoxaemia is often associated with increased bronchospasm, bronchial reactivity and anxiety, all of which can be ameliorated with oxygen therapy.

The initial effect of β-agonist medication in an acute asthma attack is to cause the pulmonary vasculature to dilate which tends to exacerbate the oedema. This results in a further deterioration in the V/Q mismatching and hypoxaemia. In an acute asthma attack, β-agonist medication should generally always be given with oxygen.

A helium-oxygen gas mixture for use in obstructive airways disease is known. The usefulness of helium is related to its physical properties. It is an inert gas without any pharmacological activity. It has a lower density than nitrogen or oxygen such that when helium is mixed with oxygen this results in lower airways resistance than either oxygen alone or an oxygen nitrogen mixture. The Reynold's number is reduced such that areas of turbulent flow in the inflamed airways are converted to laminar flow.

In the distal airways, the low solubility of helium prevents any significant absorption into the pulmonary vasculature. This prevents atelectasis formation that can occur with 100% oxygen (peripheral lung collapse). There is laminar flow in the distal airways that is dependent on the viscosity of the inhaled gas. Although helium is relatively viscous compared to oxygen or nitrogen, there is commonly a net gain in gas flow overall in acute asthma using a helium oxygen mix.

A helium:oxygen mixture of 80:20 has been shown to reduce pulses paradoxus and increase peak expiratory flow in patients with acute asthma. This reduces muscle fatigue, maintains arterial oxygenation and keeps the patient in good conditions until other forms of medication have had the opportunity to exert their effect.

An additional benefit of a helium oxygen mixture is that when administered to patients with an acute myocardial infarction the myocardium appears to be stablised reducing the risk of ventricular arrhythmias.

Currently, oxygen is administered under medical supervision. There is a need for patients, for example asthmatics during an attack, to be able to self-administer oxygen so as to optimise their treatment. However, for patients with chronic obstructive airway disease, high partial pressures of oxygen can compromise their hypoxic drive.

The paper "Site of Action of Inhaled 6 Per Cent Carbon Dioxide in the Lungs of Asthmatic Subjects before and after Exercise", by H K Fisher et al, American Review of Respiratory Disease, Volume 114 (1976), pp 861-870 discloses that the use of air enriched to 6% by volume in carbon dioxide assists the breathing of asthmatic patients.

The paper "Helium-Oxygen Mixtures in Intubated Patients with Status Asthmaticus and Respiratory Acidosis" by E H Gluck et al, Chest, Volume 98, No. 3, September 1990, pp 693 to 698, discloses ventilating patients with a mixture of helium and oxygen containing 60% by volume of helium and 40% by volume of oxygen.

The paper "Intérêt médical des mélanges respiratoires Oxygène-Hélium (Héliox), by L Barthelemy et al, Rev. Pneumol. Clin. (1993) Vol 49, pp 92-98, discloses that helium-oxygen mixtures may be used as therapeutic agent in the treatment of acute respiratory distress or severe disorders of ventilation caused by obstruction.

The paper "Melange hélium-oxygène. Prise en charge prehospitalière des asthmes aigus graves. Étude prospective: SAMU 69 (Octobre 1991 - décembre 1992) discloses the use of helium-oxygen mixtures in the treatment of asthma.

GB-A-1 396 772 discloses a gas mixture containing 40 to 60% by volume of nitrous oxide, 3 to 10% by volume of carbon dioxide and 30 to 55% by volume of oxygen, the proportion by volume of oxygen being at least 5 times that of carbon dioxide, the gas mixture having analgesic properties.

There is therefore a need for a gas mixture which can be made readily available to all patients for self administration, for example at the onset of an acute asthma attack or for general medical use, and which can overcome the above difficulties.

In accordance with the invention, there is provided a medical gas mixture which comprises from 25 to 35% oxygen, from 2 to 10% carbon dioxide and the balance, except for incidental constituents not adversely affecting the basic properties of the gas mixture, being helium. Unless otherwise stated, all gas percentages in this specification and in the claims are by volume.

Preferably, the mixture contains from 30 to 35% for the most effective treatment of asthma in particular.

Preferably also, the mixture contains from 3 to 7% carbon dioxide and most preferably does not exceed 5%.

Although it is known that the administration of a small amount of carbon dioxide has a number of useful pharmacological actions, for example it is a potent stimulus for respiration and the natural presence in a person's exhaled breath performs that action, its role in the stated amounts in the gas mixture of the invention has been shown to be especially critical. Carbon dioxide is also a bronchodilator.

With regard to helium, this is preferably present in the gas mixture in an amount of at least 60% in order in particular to achieve optimum flow dynamics in the patient to be improved. Preferably the helium content of the gas mixture is at least 65%.

An example of an advantageous gas mixture of the invention is one containing 33 to 35% oxygen 3 to 5% carbon dioxide and the balance helium.

## Claims

1. A medical gas mixture which comprises from 25 to 35% by volume oxygen, from 2 to 10% by volume carbon dioxide and the balance, except for incidental constituents not adversely affecting the basic properties of the gas mixture, being helium.

2. A medical gas mixture according to Claim 1 containing from 30 to 35% by volume oxygen.

3. A medical gas mixture according to claim 1 or claim 2 containing from 3 to 7% by volume carbon dioxide.

4. A medical gas mixture according to Claim 3 containing no more than 5% by volume carbon dioxide.

5. A medical gas mixture according to any preceding claim containing at least 60% by volume helium.

6. A medical gas mixture according to Claim 5 containing at least 65% by volume helium.

7. A medical gas mixture according to Claim 1 containing from 33 to 35% by volume oxygen, 3 to 5 % by volume carbon dioxide and the balance helium.

## Patentansprüche

1. Medizinisches Gasgemisch, das 25 bis 35 Vol.-% Sauerstoff, 2 bis 10 Vol.-% Kohlendioxid und im übrigen mit Ausnahme zufälliger, die grundsätzlichen Eigenschaften des Gasgemischs nicht ungünstig beeinflussender Bestandteile Helium enthält.

2. Medizinisches Gasgemisch nach Anspruch 1 mit 30 bis 35 Vol.-% Sauerstoff.

3. Medizinisches Gasgemisch nach Anspruch 1 oder Anspruch 2 mit 3 bis 7 Vol.-% Kohlendioxid.

4. Medizinisches Gasgemisch nach Anspruch 3 mit nicht mehr als 5 Vol.-% Kohlendioxid.

5. Medizinisches Gasgemisch nach einem der vorhergehenden Ansprüche, mit mindestens 60 Vol.-% Helium.

6. Medizinisches Gasgemisch nach Anspruch 5 mit mindestens 65 Vol.-% Helium.

7. Medizinisches Gasgemisch nach Anspruch 1 mit 33 bis 35 Vol.-% Sauerstoff, 3 bis 5 Vol.-% Kohlendioxid und im übrigen Helium.

## Revendications

1. Mélange gazeux à usage médical, qui comprend de 25 à 35 % d'oxygène en volume, de 2 à 10 % de dioxyde de carbone en volume, le reste, à l'exception de constituants accessoires n'affectant pas négativement les propriétés de base du mélange gazeux, étant de l'hélium.

2. Mélange gazeux à usage médical selon la revendication 1, contenant de 30 à 35 % d'oxygène en volume.

3. Mélange gazeux à usage médical selon la revendication 1 ou la revendication 2, contenant de 3 à 7 % de dioxyde de carbone en volume.

4. Mélange gazeux à usage médical selon la revendication 3, ne contenant pas plus de 5 % de dioxyde de carbone en volume.

5. Mélange gazeux à usage médical selon l'une quelconque des revendications précédentes, contenant au moins 60 % d'hélium en volume.

6. Mélange gazeux à usage médical selon la revendication 5, contenant au moins 65 % d'hélium en volume.

7. Mélange gazeux à usage médical selon la revendication 1, contenant de 33 à 35 % d'oxygène en volume, 3 à 5 % de dioxyde de carbone en volume, le reste étant de l'hélium.
